# EUROPEAN PATENT SPECIFICATION

(11) **EP 1 007 082 B1**
(45) Date of publication and mention of the grant of the patent: **18.10.2006**
(21) Application number: 98904563.8
(22) Date of filing: 15.01.1998
(51) Int. Cl.: A61K 38/19, C07K 14/525

(54) **MODIFIED TUMOR NECROSIS FACTOR**
MODIFIZIERTER TUMOR-NEKROSEFAKTOR
FACTEUR DE NECROSE DES TUMEURS MODIFIE

(30) Priority: 15.01.1997 US 35521 P; 14.01.1998 US 6810
(43) Date of publication of application: 14.06.2000
(73) Proprietor: Phoenix Pharmacologics, Inc., Exton, PA 19341 (US)
(72) Inventor: CLARK, Mike, A., Lexington, KY 40502 (US)
(74) Representative: von Füner, Nicolai
(86) International application number: PCT/US1998/000683
(87) International publication number: WO 1998/031383

(56) References cited:
- WO-A-97/32607
- US-A- 4 640 835
- US-A- 5 264 209
- US-A- 5 695 760
- TSUTSUMI Y ET AL: "MOLECULAR DESIGN OF HYBRID TUMOUR NECROSIS FACTOR-ALPHA II: THE MOLECULAR SIZE OF POLYETHYLENE GLYCOL-MODIFIED TUMOUR NECROSIS FACTOR-ALPHA AFFECTS ITS ANTI-TUMOR POTENCY" BRITISH JOURNAL OF CANCER, LONDON, GB, vol. 74, no. 7, October 1996 (1996-10), pages 1090-1095, XP009006144 ISSN: 0007-0920
- TSUTSUMI Y ET AL: "MOLECULAR DESIGN OF HYBRID TUMOR NECROSIS FACTOR-ALPHA III: POLYETHYLENE GLYCOL-MODIFIED TUMOR NECROSIS FACTOR-ALPHA HAS MARKEDLY ENHANCED ANTITUMOR POTENCY DUE TO LONGER PLASMA HALF-LIFE AND HIGHER TUMOR ACCUMULATION" JOURNAL OF PHARMACOLOGY AND EXPERIMENTAL THERAPEUTICS, AMERICAN SOCIETY FOR PHARMACOLOGY AND, US, vol. 278, no. 3, 1996, pages 1006-1011, XP001010039 ISSN: 0022-3565
- TSUTSUMI Y ET AL: "MOLECULAR DESIGN OF HYBRID TUMOUR NECROSIS FACTOR ALPHA WITH POLYETHYLENE GLYCOL INCREASES ITS ANTI-TUMOUR POTENCY" BRITISH JOURNAL OF CANCER, LONDON, GB, vol. 71, no. 5, 1995, pages 963-968, XP009006142 ISSN: 0007-0920
- JAP. J. CANCER RES., January 1994, Vol. 85, No. 1, TSUTSUMI et al., "Chemical Modification of Natural Human Tumor Necrosis Factor alpha with Polyethylene Glycol Increases Its Anti-Tumor Potency", pages 9-12, XP002913448
- NATURE, 27 December 1984, Vol. 312, PENNICA et al., "Human Tumor Necrosis Factor: Precursor Structure, Expression and Homology to Lymphotoxin", pages 724-729, XP002913449

## Description

This invention is directed, *inter alia,* to tumor necrosis factor modified with polyethylene glycol having a molecular weight in the range of 20,000 to 40,000 and the use of a therapeutically effective amount of the modified TNF for the preparation of a pharmaceutical composition for treating tumors.

Malignant melanoma (stage 3) is a fatal disease killing most patients within one year of diagnosis. The incidence of melanoma is rapidly increasing in the United States and is even higher in other countries, such as Australia. Effective treatments for patients suffering from melanoma are urgently needed.

Kidney cancer currently kills approximately 13,000 individuals in the United States each year. This form of cancer is frequently not detected until it is well advanced. The only form of treatment that significantly affects a patient's prognosis is surgical resection of the affected organ. Unfortunately, because this type of cancer is highly metastatic, complete removal of all the metastasis is difficult, if not impossible.

Colon cancer is one of the most prevalent forms of cancer and currently kills approximately 140,000 individuals in the United States each year. Although there have been a large number of traditional chemotherapeutic drugs developed to treat this disease, long term survival (defined as the percentage of patients surviving five years or more) has not appreciably changed in the last four decades. Furthermore, all of the traditional chemotherapeutic drugs developed are highly toxic, have deleterious and often fatal side effects, and are expensive. A curative, non-toxic treatment for this disease is urgently needed.

A hallmark of melanomas, kidney and colon tumors is that these tumors quickly develop resistance to traditional chemotherapies. Even though patients may initially respond to chemotherapeutic treatment, drug-resistant tumors quickly develop and ultimately kill the patient. An alternative way to treat these tumors would be to identify an "Achilles Heel" in the tumors and to develop therapies that would selectively treat that target. One such potential target has been identified. Specifically, it has been noted that all three of these types of tumors require extensive vascularization of each of the metastases in order for the cancers to grow. Therefore, one would predict that a therapeutic agent which would inhibit the vascularization of these tumors may provide a unique means of treating these tumors.

Tumor necrosis factor (TNF) is one of many cytokines, which are a group of diverse proteins produced by leukocytes and related cells, having immunostimulating activity. TNF was originally named for its ability to cause tumor necrosis. There are at least two different mechanisms by which TNF is believed to kill tumors. The first is by a direct effect on the tumor itself. Alternatively, TNF can selectively disrupt the vascularization of tumors. In an early manuscript describing TNF, Carswell and Old reported that the METH A tumor cells were completely resistant to TNF *in vitro. J. Proc. Natl. Acad. Sci USA,* **72**:3666-3670 (1975). However, METH A tumors in mice were extremely sensitive to TNF *in vivo.* This was believed to be because TNF selectively disrupted the vascularization of these tumors. It was later shown that some as-yet-unidentified factor is released by some tumors that renders the normal vascular endothelial cells adjacent to them susceptible to TNF killing. In short, TNF kills these tumors not by directly killing the tumor cells, but rather by killing the normal vascular endothelial cells that line the blood vessels that provide the tumor with blood, oxygen and other nutrients necessary to live and grow.

Unfortunately, early clinical trials attempted to develop TNF as a direct tumoricidal agent. When used in this fashion, large doses of TNF needed to be injected as it quickly (less than 20 minutes) leaves the circulation. Furthermore, these high doses of TNF induced "shock"-like symptoms characterized by a precipitous drop in blood pressure. An alternative way of using TNF would be to formulate it so that it remains in the circulation. By doing this, the TNF would have more time to interact with the vasculature of the tumor and would have sufficient time to disrupt the blood supply to the tumor.

Several other therapeutic proteins have been formulated with polyethylene glycol (PEG) so that they would circulate longer and remain in the vasculature. These proteins include asparaginase, adenosine deaminase, and super oxide dismutase. See, for example, Harras, J.M., in "Polyethylene Glycol Chemistry: Biotechnical and Biochemical Applications," Plenum Press (1992). A group of Japanese investigators have previously described that TNF could be formulated with certain PEG and that the resulting material had substantially increased circulating half-life and greater anti-tumor activity. Tsutsumi, Y., et al., *Jap. J. Cancer Res.,* **85:**9-12 (1994); Tstutsumi, Y., et al., *Jap. J. Cancer Res.,* **85:**1185-1188 (1994); Tsutsumi, Y., et al., *Jap. J. Cancer Res.,* **87:**1078-1085 (1997). These investigators used only PEG with a molecular weight of 5000 coupled to primary amines on TNF with a succinimidyl succinate linker.

It has now been found, surprisingly, that modifying TNF with polyethylenegycol (PEG) having an approximate weight average molecular weight in the range of 20,000 to 40,000, preferably in the range of 20,000 to 30,000, significantly increases the circulating half-life of the TNF and also enhances the tumoricidal activity of the TNF. For example, the serum half life of TNF has been increased, by such modification, from as little as 20 minutes up to fifteen days, and the anti-tumor ED50 has been decreased from as much as 1000-3000 IU to as little as 10-50 IU. As a result of the modification, lower doses of the TNF may be administered to effectively treat tumors, with fewer, accompanying adverse side effects to the patient.

This invention, therefore, relates to modified TNF, wherein said modification comprises covalently bonding to said TNF, either directly or through a biocompatible linking agent, one or more PEG molecules having an approximate weight average molecular weight in the range of 20,000 to : 40,000. The TNF is modified with five to twelve of the PEG molecules, preferably, with about five to nine PEG molecules.

This invention also relates to a use of a therapeutically effective amount of the modified TNF for the preparation of a pharmaceutical composition for treating a patient suffering from a tumor.

This invention further relates to a method of enhancing the circulating half life of TNF comprising modifying said TNF by covalently bonding to it between about five and twelve PEG molecules having an approximate weight average molecular weight in the range of- 2 0,000 to 40,000.

This invention further relates to a method of enhancing the tumoricidal activity of TNF comprising modifying said TNF by covalently bonding to it between about five and twelve PEG molecules having an approximate weight average molecular weight in the range of 20,000 to 40,000.
Figure 1 is a graph depicting the circulating half life in mouse serum of native TNF-α (open circles), SS 5,000 MW PEG-TNF-α (closed circles), and 20,000 MW PEG-TNF-α (open triangles).
Figure 2 is a graph depicting the circulating half life in mouse serum of native TNF-α (open circles), SS 5,000 MW PEG-TNF-α (closed circles), SS 12,000 MW PEG-TNF-α (closed triangles), SS-20,000 MW PEG-TNF-α (open triangles), NHS 12,000 MW PEG-TNF-α (closed squares), and NHS 20,000 MW PEG-TNF-α (open squares).

"Tumor necrosis factor" or "TNF" as used herein encompasses either naturally derived protein, such as isolated human or mouse TNF proteins, or protein produced using recombinant technology, such as recombinant murine TNF and recombinant human TNF. Although the TNF-α protein is preferred, the term "TNF" also encompasses TNF-β protein. The terms also encompass TNF proteins that have been mutated by deletion or alteration of amino acids without significantly impairing biological activity (e.g., as non-limiting examples, delete amino acids 212-220 or change lysine at 248, 592, 508 to alanine).

"Polyethylene glycol" or "PEG" refers to mixtures of condensation polymers of ethylene oxide and water, in a branched or straight chain, represented by the general formula H(OCH₂CH₂)ₙOH. "Polyethylene glycol" or "PEG" is used in combination with a numeric suffix to indicate the approximate weight average molecular weight thereof. For example, PEG 5,000 refers to polyethylene glycol having an approximate weight average molecular weight of about 5,000; PEG 12,000 refers to polyethylene glycol having an approximate weight average molecular weight of about 12,000; and PEG 20,000 refers to polyethylene glycol having an approximate weight average molecular weight of about 20,000. Such polyethylene glycols are available from several commercial sources, and are routinely referred to, as indicated above, by their weight average molecular weights.

"Melanoma" may be a malignant or benign tumor arising from the melanocytic system of the skin and other organs, including the oral cavity, esophagus, anal canal, vagina, leptomeningers, and/or the conjunctivae or eye. The term "melanoma" includes, for example, acral-lentginous melanoma, amelanotic melanoma, benign juvenile melanome, lentigo maligna melanoma, malignant melanoma, nodular melanoma, subungual melanoma and superficial spreading melanoma.

"Patient" refers to an animal, preferably a mammal, more preferably a human.

"Biocompatible" refers to materials or compounds which are generally not injurious to biological functions and which will not result in any degree of unacceptable toxicity, including allergenic and disease states.

"Circulating half life" refers to the period of time, after injection of the modified TNF into a patient, until a quantity of the TNF has been cleared to levels one half of the original peak serum level. Circulating half life may be determined in any relevant species, including humans or mice.

"Covalently bound" as used herein refers to a covalent bond linking the TNF protein to the PEG molecule, either directly or through a linker.

According to this invention, TNF is modified with polyethylene glycol having an approximate weight average molecular weight in the range of 20,000 to 40,000, preferably in the range of 20,000 to 30,000. Generally, polyethylene glycol with a molecular weight of 30,000 or more is difficult to dissolve, and yields of the formulated product are greatly reduced. The polyethylene glycol may be branched or straight chain, but is preferably a straight chain.

The polyethylene glycols may be bonded to the TNF through biocompatible linking groups. As discussed above, "biocompatible" indicates that the compound or group is non-toxic and may be utilized *in vitro* or *in vivo* without causing injury, sickness, disease or death. PEG may be bonded to the linking group, for example, via an ether bond, an ester bond, a thiol bond, or an amide bond. Suitable biocompatible linking groups include, for example, an ester group, an amide group, an imide group, a carbamate group, a carboxyl group, a hydroxyl group, a carbohydrate, a maleimide group (including, for example, succinimidyl succinate (SS), succinimidyl propionate (SPA), succinimidyl carboxymethylate (SCM), succinimidyl succinamide (SSA), or N-hydroxy succinimide (NHS), an epoxide group, an oxycarbonylimidazole group (including, for example, nitrophenyl carbonate (NPC) or trichlorophenyl carbonate (TPC)), a trysylate group, an aldehyde group, an isocyante group, a vinylsulfone group, a tyrosine group, a cysteine group, a histidine group or a primary amine. Preferably, the biocompatible linking group is an ester group and/or a maleimide group and bonds to the TNF through a primary amine on the TNF protein. More preferably, the linking group is SS, SPA, SCM, SSA or NHS; with SS being the most preferred.

Alternatively, TNF may be coupled directly to PEG (i.e., without a linking group) through an amino group, a sulfhydryl group, a hydroxyl group, or a carboxyl group.

Methods for covalently bonding TNF to PEG, directly or via a biocompatible linking group, are known in the art, as described, for example, in Harras, J.M., in "Polyethylene Glycol Chemistry: Biotechnical and Biochemical Applications," Plenum Press (1992), the disclosure of which is herein incorporated by reference. It is preferred that the TNF protein be covalently bonded to five to twelve PEG molecules. Methods for determining the number of PEG molecules bonded to the protein are known in the art, for example, Habeeb, A.F.S.A., *Anal. Biochem.,* **14**:328-339 (1966); Harras, J.M., *supra.,* herein incorporated by reference. The number of PEG molecules bonded to TNF will vary according to the linking group utilized, the length of reaction, and the molar ratios of TNF and PEG utilized in the reaction.

As one skilled in the art would recognize, the modified TNF of this invention may be administered in a number of ways, for example, orally, intranasally, intraperitoneally, parenterally, intravenously, intralymphatically, intratumorly, intramuscularly, interstitally, intrarterially, subcutaneously, intraocularly, intrasynoially, transepithelially, and transdermally. A therapeutically effective amount of one of the modified compounds of the present invention is an amount effective to inhibit tumor growth, and that amount may vary according to the method of administration. Generally, effective doses should be in the range of about 0.001 to 0.1 mg/kg, once a week. The modified TNF may be formulated with pharmaceutically acceptable carriers and diluents, as known in the art. For example, for intravenous? administration, the modified TNF may be mixed with a phosphate buffered saline solution, or any other appropriate solution known to those skilled in the art, prior to injection. Tests have shown that the modified TNF is particularly effective in treating melanoma, colon cancer, kidney cancer and breast cancer tumors.

The invention is further demonstrated in the following examples, which are for purposes of illustration, and are not intended to limit the scope of the present invention.

TNF used in the experiments described below was of mouse and human origin. The human TNF (whole protein) was produced in *E. coli,* and murine TNF as well as the human TNF mutants were produced in *Pichea pastoris.* Recombinant TNF was produced in *E. coli* or *Pichea* using methods similar to those described in Pennica, D., et al., *Nature,* **312:**724-729 (1981); Streekishna, K. , et al., *Biochemistry,* **28:**4117-4125 (1989). The mouse TNF was produced in *E. coli* and in *Pichea.*

### Example 1

### Specific Activity of TNF-α

Prior to pegylation, mature tissue necrosis factor (TNF-α) (human recombinant) was tested using a L929 cytotoxicity assay as described below. The specific activity of the TNF-α was 10⁶ I.U. units per milligram. Densitometry of an SDS-PAGE gel showed that the material was 99% pure.

The material (1 ml at 16.8 mg/ml, determined by the method of Bradford, M.M., *Anal. Biochem.,* **72**:248-254 (1976), with a bovine serum albumin (BSA) standard, Peterson modification) was concentrated to approximately 0.1 ml using a 3,000 kDa cutoff Centricon. This material was then diluted to 4 ml with 100 mM phosphate buffer, pH 8.0 and reconcentrated to 0.1 ml. This procedure was repeated twice, and the resulting material was finally collected in a total volume of 1 ml of the same buffer.

The protein concentration was then determined by the method of Bradford, above. BSA was used as a standard, and about 0.6 mg of protein was recovered.

### Pegylation

PEGylating reactions were performed using the general methods described in Harras, J.M., cited above. To TNF (1mg/ml in 100 mm phosphate buffer, pH 7.2-7.5), the SS-PEG or NHS-PEG? was added at a 10 to 50 molar excess and mixed for one hour at room temperature. The specific pH and molar ratio used varied with the reactivity of the PEG and had to be empirically determined. The YEG-TNF was separated from unreacted PEG and TNF by ultra filtration using a 100 kDa cut off filter. In each of the modifications referenced in this example, the TNF was modified with five to nine molecules of PEG.

Purity of the PEG-TNF was assessed by SDS-PAGE and the percent of primary amines modified by this procedure was determined using florescamine as described by S.J. Stocks (Anal. Biochem. **154:**232 (1986)). SDS-PAGE results indicated that very little, if any, native TNF-α remained in the preparation after pegylation.

### In vitro activity of the PEG-TAT-α

The PEG-TNF-α were examined for in vitro cytotoxic activity using the L-929 cytotoxicity assay performed according to the procedure set forth below, with the exception that the passage number of the cells was not known. The specific activity of the TNF-α starting material was 1.5 x 10⁶ units/mg or about one half of the original specific activity measurement. The differences in specific activity were attributed to the use of a different protein determination method and the unknown passage number of the cells.

The specific activity of the SS 5,000 MW PEG-TNF-α was 0.7 x 10⁶ units/mg, or about one half the specific activity of the native material. The specific activity of the SS-20,000 MW PEG-TNF-α was 0.8 x 10⁶ units/mg, a value similar to the SS 5,000 MW PEG-TNF-α.

### Lethality of the PEG-TNF-α

As a screen, two C57 b16 mice (female, 20-25 g) were injected intraperitoneally (i.p.) with either native TNF-α or SS-PEG-TNF-α and survival of the animals was monitored. The doses used were 1, 5, and 10 thousand units of activity.

With native TNF-α, the following results were obtained:
10,000 I.U. - both mice dead the next morning
5,000 I.U. - one mouse dead next morning; the second mouse in obvious distress (hair ruffled and little movement) and dead after 2 days
1,000 I.U. - one mouse dead the next morning; the second mouse in distress (hair ruffled and little movement) and in such poor condition after 2 days that it was euthanized

With the SS-PEG-TNF-α, all mice at all doses remained in good health for two weeks following injection. Behavior was normal, as was eating and drinking. There was no change in coat (fur was not ruffled). All of the mice were euthanized 15 days post-injection.

### Determination of serum half life of PEG-TNF-α

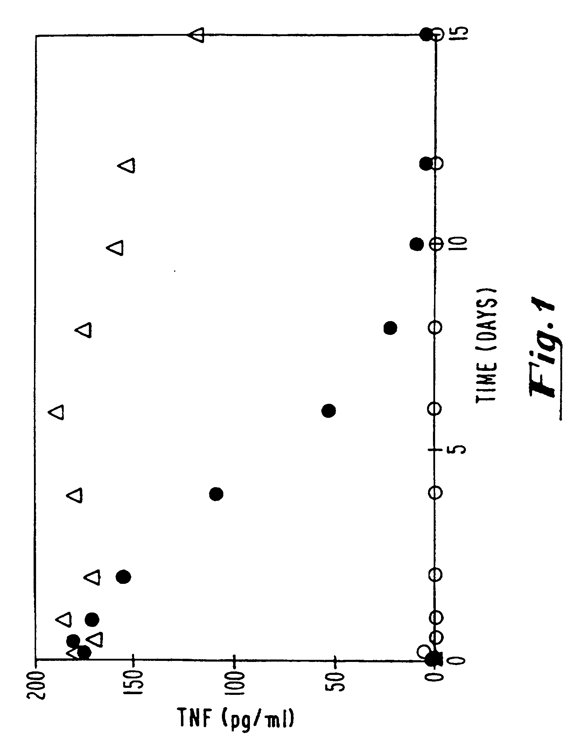

An ELISA assay for human TNF obtained from Genzyme was used. The kit was used as suggested by the manufacturer. Mice were injected with either TNF-α or PEG-α (100 units) i.p., and approximately 25 µl of serum was collected from retro-orbital bleeds at the times indicated in Fig. 1. A total of 5 mice (female, C57 b16 mice, 20-25g) were in each group.

The native TNF-α ( open circles) was cleared very fast, and the only data point above baseline was 30 minutes post-injection.

The SS 5,000 MW peg-TNF-α (closed circles) had a half life of about 4 days. The half life of the 20,000 MW PEG-TNF-α (open triangles) was > 15 days.

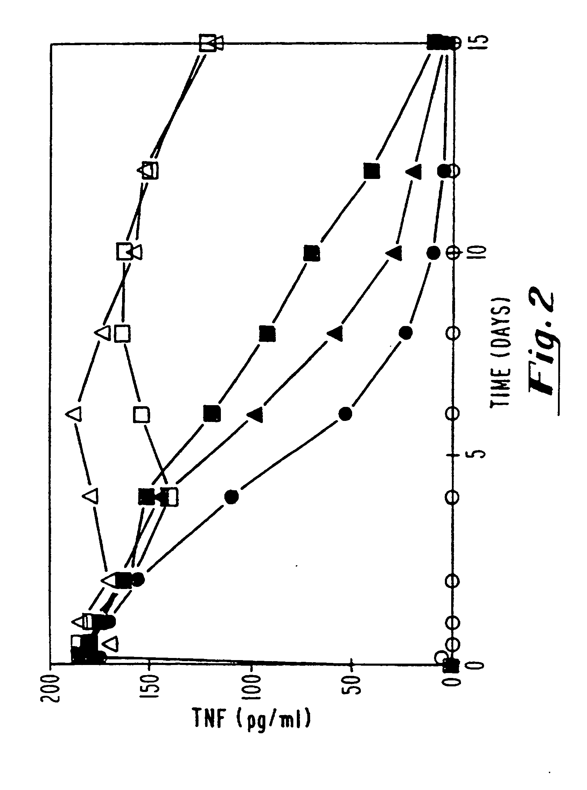

This experiment was repeated using the treatment groups listed below, and the results presented in Fig. 2: native TNF-α (open circles); SS 5,000 MW PEG-TNF-α (closed triangles); SS 20,000 MW PEG-TNF-α (open triangles); NHS 12,000 MW PEG-TNF-α (closed squares). The serum half life for the different treatment groups was > 15 days for NHS 20,000 MW PEG-TNF-α and SS 20,000 MW PEG-TNF-α; approximately 4 days for SS 5,000 MW PEG-TNF-α; approximately 6 days for SS 12,000 MW PEG-TNF-α; approximately 8 days for NHS 12,000 MW PEG-TNF-α; and 30 min post-injection for native TNF-α. In summary, each PEG-TNF-α exhibited a much longer half life than native TNF-α; however, the NHS 20,000 MW PEG-TNF-α and the SS 20,000 MW PEG-TNF-α had significantly longer half lives (> 15 days) than the TNF-α modified with lower molecular weight PEG.

### Antitumor Activity of PEG-TNF-α

The B16 melanoma model was used. C57 b16 mice (female, 20-25 g) were injected with one million B16 cells, s.q. on flank. The tumors were allowed to grow for one week before beginning treatment. Each treatment group consisted of 5 mice.

The treatment groups included: phosphate buffer control (ph 7.5), native TNF-α ( 10 I.U. and 100 I.U.) in phosphate buffer (ph 7.5), and SS-PEG-TNF-α (10 I.U., 100 I.U. and 1000 I.U.) in phosphate buffer (ph 7.5). The mice were injected with 0.1 ml i.p. once a week on Day 7, Day 14, and Day 21. Survival of the animals was recorded.

After Week 2 (one week after first treatment), the tumors in the control animals were quite visible. None of the SS-PEG-TNF-α treated animals appeared to have tumors growing.

At Day 22, the results presented in Table 1 were obtained:

**Table 1**

| **Treatment Group** | | **Results** |
|---|---|---|
| **Control** | | 4 dead, 1 with large tumor |
| **Native TNF-α** | | |
| | 10 I.U. | all animals dead |
| | 100 I.U. | 4 dead, 1 with large tumor |

| **5000 MW SS-PEG-TNF-α** | | |
|---|---|---|
| | 10 I.U. | 2 dead, 3 with large tumors |
| | 100 I.U. | 2 dead, 2 with tumors, I tumor-free |
| | 1000 I.U. | 1 dead, 3 with small tumors, 1 tumor-free |

| **20000 MW SS-PEG-TNF-α** | | |
|---|---|---|
| | 10 I.U. | 0 dead, 1 with small tumor, 4 tumor-free |
| | 100 I.U. | all animals tumor-free |
| | 1000 I.U. | all animals tumor-free |
| | | |

After Day 180, the results presented in Table 2 were obtained.

**Table 2**

| **Treatment Group** | | **Survival Time** | **Average** |
|---|---|---|---|
| **Control** | | 18, 18, 20, 21, 24 days | 20.2 days |
| **Native TNF-α** | | | |
| | 10 I.U. | 17, 18, 19, 21, 21 days | 20.2 days |
| | 100 I.U. | 16, 18, 19, 19, 23 days | 19.0 days |
| **5000 MW SS-PEG-TNF-α** | | | |
| | 10 I.U. | 20, 22, 24, 26, 27 days | 23.6 days |
| | 100 I.U. | 21, 22, 24, 26, 27 days | 35.0 days |
| | 1000 I.U. | 21, 49, 53 days; 2 animals alive | |
| **20,000 MW SS-PEG-TNF-α** | | | |
| | 10 I.U. | 38 days, 4 animals alive | |
| | 100 I.U. | all animals alive | |
| | 1000 I.U. | all animals alive | |

These results indicate that modification of TNF with PEG not only reduces the lethality of the TNF but that the TNF modified with PEG having a molecular weight of approximately 20,000 exhibited surprisingly enhanced circulating half lives and surprisingly and significantly enhanced anti-tumor activity.

These results are surprising for a number of reasons. There was no way to predict that modifying TNF with high molecular weight PEG would increase the circulating half-life of the TNF. Indeed, the clearance rate of proteins in general cannot be predicted based on their molecular weight. That modification of TNF with high molecular weight PEG not only does not diminish, but actually enhances, the tumoricidal activity of the TNF is surprising in view of the added stearic hindrance expected to be created by the high molecular weight modifier. Still further, one would have predicted that the modified TNF, because of its enhanced circulating half life, would have been even more toxic than the native TNF, which was not the case.

Following is a description of the cytotoxicity assay utilized in this example.

### A. Materials

L929 fibroblasts ATCC #CCL1 NCTC clone 929.
Dulbecco's Modified Essential Medium (DMEM)
Fetal Bovine Serum (GIBCO Laboratories, Grand Island, NY #16000-010) HEPES Buffer 1M in Normal Saline (BioWhittaker, Inc. #17-737E)
Gentamicin Sulfate (BioWhittaker, Inc., #17-518Z)
Trypsin-EDTA 1X (GIBCO Laboratories, #25300-021)
Trypsin Blue Stain (GIBCO Laboratories, #15250-012)
Tissue culture flasks, 150cm², 75cm², 25cm² (Coming, Inc., Corning, NY, #25120, #25110, #25100)
96-Well cell culture plates, flat bottom (Corning Inc., #25861)
12-Channel pipetter (Titertek)
Sterile tips for pipetter (Intermountain Scientific Corp. , Bountiful, UT, #P-3250-8)
Sterile reagent reservoirs (Costar Corp., Cambridge, MA, #4870)
Recombinant Human Tumor Necrosis Factor-α (TNF-α) (prepared in-house)
Albumin, bovine, 10% in IMEM (Boehringer Mannheim Corp., Indianapolis, IN, #652237)
Phosphate Suffered Saline (PBS)
Dulbecco's Phosphate Buffered Saline (D PBS) (GIBCO Laboratories, #310-4190)
Microtiter Plate Reader (Molecular Devices Corp., Menlo Park, CA, Emax)

### B. Propagation of L929 Fibroblasts:

1. Maintain fibroblasts by passing twice weekly in DMEM/10% FBS as follows. Aspirate the medium from the flask, leaving the adherent cells. Wash the cell monolayer with 5 ml trypsin-EDTA and aspirate off the trypsin-EDTA. Incubate 1 to 2 minutes at 37°C. Add 15 ml DMEM/FBS to wash off the monolayer and stop the enzymatic activity of the trypsin.
2. Count the cells and evaluate viability by Trypan Blue exclusion. Inoculate 30 ml DMEM/FMS without antibiotics in a T75 flask with 1 X 10⁶ cells. Incubate in a 37°C, 5% CO₂ humidified incubator.
3. For the assay, inoculate 60 ml DMEM/FBS in a T150 Flask with 2 X 10° cells. Incubate flask for 3 or 4 days until the cells approach confluency (80-90%) **C. TNF-α In Vitro Cytotoxicity Assay**

### Day One: Preparing the cells

1. Trypsinize cells and dilute with DMEM/FBS to a final concentration of 1.22 X 10⁶ cells/ml. Add gentamicin sulfate to a final concentration of 50 µg/ml. A cell count of 2 X 10⁶ cells are required for each plate, and 1.75-2 X 10⁷ cells can be expected from a T150 flask.
2. Plate the L929 fibroblasts by adding 150 µl of the cell suspension to each well of a 96-well flat-bottom tissue culture plate. Incubate overnight in a 37°C, 5% CO₂ humidified incubator.

### Day Two: Adding the samples

1. Using an inverted microscope, check the confluency of each well before proceeding. Each well must be equivalently confluent for the assay to be reproducible. The outside wells of the plate are not used for assay calculations due to the uneven growth of the cells in these wells. The cells must be 75 to 90% confluent for best sensitivity. As the passage number of the cells increases, the hours per doubling of the cells decreases. The cell number/well can be adjusted accordingly to obtain the correct confluency.
2. Prepare TNF-α standard working solution. Keep at 4°C until use.
3. Add 150 µl of the TNF working solution to the first well of columns 2 and 3. Add 150 µl of sample to the first well of Columns 4 through 10. Add DMEM/FBS/gentamicin to Columns 1, 11, and 12. Make 2-fold serial dilutions by mixing the contents of the first wells and transferring 150 µl to the second wells of the columns, using a 12-channel pipetter. Finally, mix the contents of the 8th wells (row H) and discard 150 µl form each well. At this point, all wills should contain 150 µl.
4. Incubate the plates 20 hours in a 37°C, 5% CO₂ humidified incubator.

### Day three:

Viability of the cells was determined by adding 20 µl of 3[4,5-dimethylthiazol-2-yl]-2,5-diphenyltetrazolium bromide (MTT) (25 mg/ml in phosphate buffered saline pH 7.4) to each well of the culture plate and incubating the cultures at 37°C for four hours. After that time, the culture supematents were discarded and 150 µl of DMSO was added to each well. The absorbance of each well at 570 nm was determined using a micro titer plate reader. Wells that exhibit an A₅₄₀ closest to 50% of the arithmetic mean of the control are considered to represent 50% lysis (1 unit) of the L929 cells.

### D. SOLUTIONS

### TNF-α Standard (10 µg)

Thaw frozen TNF-α standard on ice. Aliquot into screw cap cryotubes at 2 µg/tube and store at -80°C.

### TNF-α Standard Stock Solution (256 ng/ml)

To one tube (2µg) of TNF-α standard, add 7.81 ml 1% albumin in D-PBS/gentamicin. Aliquot into 1 ml tubes and keep at 4°C. Solution is typically used for six months.

### TNF-α Working solution (0.8 ng/ml)

For two plates, add 3.12 µl stock solution to 997 µl DMEM/FBS/gentamicin. (The standard solution will be diluted 1:2 in the first well of the multiwell plate for a final concentration of 0.4 ng/ml). Keep at 4°C until use.

### Phosphate Buffered Saline 1L (PBS, 1x)

Dissolve 0.2 g Kcl, 0.2 g YH₂PO₄, 8 g NaCl, and 1.14 g Na₂HPO₄ in 900 ml water. Adjust pH to 7.4 Q.S. to 1 L. Autoclave.

### Example 2

Additional samples of human recombinant TNF modified with PEG were prepared (according to general methods described in Harras, *Supra.)* and tested for their serum half life and for retention of specific activity. In each case, the TNF was modified with about five to nine molecules of PEG. The data (Table 3) show, surprisingly, that the TNF modified with PEG having an approximate molecular weight average of about 10000 to 40000, preferably about 20000-30000, exhibit a significantly enhanced serum half life while retaining high specific activity.

The serum half-life of the various formulations of TNF were determined using ELISA assays obtained from Genzyme (Cambridge, MA), as suggested by the manufacturer. Serum samples were collected from retro orbital plexus using heparinized 50µl capillary tubes. A pretreatment blood sample was collected just prior to i.v. injection with TNF or PEG-TNF formulations. Additional blood samples were collected at 30 minutes, 24 hours as well as 3, 7, 12 and 15 days post-treatment. The samples were centrifuged and the resulting supernatant was stored frozen at -20°C until being assayed.

**Table 3**

| **Treatment** | **Serum Half Life (days)** | **% Specific Activity Retained** |
|---|---|---|
| SS-PEG 5000 TNF-α (2 tests) | 4, 4 | 55, 58 |
| SS-PEG 12000 TNF-α (2 tests) | 8, 8 | 52, 53 |
| SS-PEG 20000 TNF-α | 16 | 56 |
| SS-PEG 30000 TNF-α | 17 | 54 |
| SS-PEG 40000 TNF-α | 17 | 55 |
| SP-PEG 5000 TNF-α | 5 | 51 |
| SP-PEG 20000 TNF-α | 8 | 53 |
| Branched chain succinimide-PEG 10000 TNF-α | 7 | 49 |
| Branched chain succinimide-PEG 20000 TNF-α | 16 | 52 |
| Branched chain succinimide-PEG 40000 TNF-α | 18 | 54 |
| | | |

### Example 3

Additional samples of PEG modified, human recombinant TNF were prepared and tested for serum half life and specific activity retention. The data presented in Table 4 show that half-life and specific activity can vary dependent upon the linker utilized.

**Table 4**

| **Treatment (#PEG's/TNF)** | **Serum Half Life (days)** | **% Specific Activity Retained** |
|---|---|---|
| SCM-PEG 5000 TNF-α (5-9 PEG's) | 5 | 54 |
| Succinimidyl ester of amino acid-PEG 5000 TNF-α (6-8 PEG's) | 4 | 55 |
| Epoxide PEG 8000 TNF-α (primary amine attachment site) (9-12 PEG's) | 6 | 38 |
| Epoxide PEG 8000 TNF-α (hydroxyl attachment site) (10-20 PEG's) | 5 | 0 |
| Glycidyl ether PEG 5000 TNF-α (15 PEG's) | 12 | 0 |
| Nitrophenyl PEG 5000 TNF-α (6-9 PEG's) | 5 | 21 |
| Trichlorophenyl carbonate PEG 5000 TNF-α (12-15 PEG's) | 5 | 11 |
| PEG tresylate 5000 TNF-α (10-12 PEG's) | 2 | 8 |
| PEG aldehyde 5000 TNF-α (1 PEG) | < 1 | 100 |
| PEG aldehyde 20000 TNF-α (1 PEG) | 2 | 100 |
| PEG isocyanate 5000 TNF-α (5-12 PEG's) | 9 | 19 |
| PEG vinylsulfone 5000 TNF-α (4 PEG's) | 3 | 12 |
| PEG maleimide 5000 TNF-α (3 PEG's) | 3 | 43 |

### Example 4

Additional tests were done to evaluate the effect of PEG-modified, recombinant human TNF on mice injected with different varieties of tumor cells. The TNF utilized in these tests was modified with SS-PEG 20000 according to a method as described Harras, *Supra.* Mice were injected with 1 x 10⁶ tumor cells and, two weeks later, were injected i.p. with the PEG-TNF once a week, for three weeks. Cure was defined as the percent of animals surviving five times longer than untreated animals. Results are presented in Table 5 and indicate that the modified TNF of this invention is effective in treating melanoma tumors, kidney tumors, colon tumors, and breast tumors.

**Table 5**

| **Tumor Type** | **Cell Line** | **Dose of PEG-TNF I.U.** | **% Cure** |
|---|---|---|---|
| Melanoma | B16 | 10 | 75 |
| | | 30 | 100 |
| | | 100 | 100 |
| Kidney | G401 | 10 | 80 |
| | | 30 | 80 |
| Colon | HT29 | 10 | 40 |
| | | 30 | 60 |
| | | 100 | 80 |
| Breast | MCF7 | 10 | 0 |
| | | 30 | 0 |
| | | 100 | 20 |
| Brain | SW1088 | 100 | 0 |
| Leukemia | L1210 | 100 | 0 |
| Hepatoma | Hep3B | 100 | 0 |

### Example 5

Tests were done to evaluate TNF from a variety of sources, modified with PEG. Results are presented in Table 6.

**Table 6**

| **TNF PROTEIN** | **SPECIFIC ACTIVITY** | **LD50** | **Hypotension ED50** | **Antitumor ED50** |
|---|---|---|---|---|
| Recom. murine TNF produced in Pichea | 2.1x10⁷ IU/mg | 20µg | 1µg | > 1000 IU |
| " - modified with SS-PEG MW 20000 | 1.0x10⁷ IU/mg | 100µg | 2µg | 20 IU |
| Recom. murine TNF produced in E. coli | 2.0x10⁷ IU/mg | 70µg | 1µg | > 3000 IU |
| " - modified with SS-PEG MW 20000 | 1.0x10⁷ IU/mg | 300µg | 4µg | 20 IU |
| Human TNF mutated by deletion of amino acids 212-220 | 2.1x10⁷ IU/mg | 60µg | 1µg | > 2000 IU |
| " - modified with SS-PEG MW 20000 | 0.9x10⁷ IU/mg | 100µg | 5µg | 10 IU |
| Human TNF mutated by changing 248, 592, 508 lysine to alanine | 1.5x10⁷ IU/mg | 300µg | 100µg | >1000 IU |
| " - modified with SS-PEG MW 20000 | 0.5x10⁷ IU/mg | > 1000µg | > 100µg | 5 IU |
| | | | | |

## Claims

1. Modified TNF, comprising TNF covalently bound to between about five and twelve PEG molecules having an approximate weight average molecular weight in the range of 20,000 and 40,000.

2. The modified TNF of Claim 1 wherein said PEG has an approximate weight average molecular weight in the range of 20,000 and 30,000.

3. The modified TNF of Claim 1 wherein said PEG is covalently bonded to said TNF through a biocompatible linker.

4. The modified TNF of Claim 1 wherein said linker is N-hydroxysuccinimidyl succinate.

5. The modified TNF of Claim 1 wherein said linker is N-succinimidyl propionate.

6. The modified TNF of Claim 1 wherein said TNF is covalently bound to about five to nine PEG molecules.

7. The modified TNF of Claim 1 wherein said TNF is covalently bound to said PEG molecules through primary amines on the TNF.

8. The modified TNF of Claim 1 wherein said TNF is TNF-α.

9. The modified TNF of Claim 1 wherein said TNF is isolated human TNF.

10. The modified TNF of Claim 1 wherein said TNF is recombinant human TNF.

11. A method of enhancing the circulating half life of TNF comprising modifying said TNF by covalently bonding to it between about five and twelve PEG molecules having an approximate weight average molecular weight in the range of 20,000 to 40,000.

12. A method of enhancing the tumoricidal activity of TNF comprising modifying said TNF by covalently bonding to it between about five and twelve PE molecules having an approximate weight average molecular weight in the range of 20,000 to 40,000.

13. The use of a therapeutically effective amount of the modified TNF of Claim 1 for the preparation of a pharmaceutical composition for treating a patient suffering from a tumor.

14. The use of Claim 13 wherein said tumor is a melanoma.

15. The use of Claim 13 wherein said tumor is a colon cancer.

16. The use of Claim 13 wherein said tumor is a kidney cancer.

17. The use of Claim 13 wherein said tumor is a breast cancer.

## Patentansprüche

1. Modifizierter TNF (Tumor-Nekrosefaktor), der den TNF an zwischen etwa fünf und zwölf PEG Moleküle, die eine Molekülmasse mit einer ungefähren durchschnittlichen Masse im Bereich von 20.000 und 40.000 aufweisen, kovalent gebunden umfasst.

2. Modifizierter TNF nach Anspruch 1, wobei das PEG eine Molekülmasse mit einer ungefähren durchschnittlichen Masse im Bereich von 20.000 und 30.000 aufweist.

3. Modifizierter TNF nach Anspruch 1, wobei das PEG an den TNF durch einen biologisch verträglichen Binder kovalent gebunden ist.

4. Modifizierter TNF nach Anspruch 1, wobei der Binder N-Hydroxysuccinimidylsuccinat ist.

5. Modifizierter TNF nach Anspruch 1, wobei der Binder N-Succinimidylpropionat ist.

6. Modifizierter TNF nach Anspruch 1, wobei der TNF an etwa fünf bis neun PEG Moleküle kovalent gebunden ist.

7. Modifizierter TNF nach Anspruch 1, wobei der TNF an die PEG Moleküle durch Primäramine auf dem TNF kovalent gebunden ist.

8. Modifizierter TNF nach Anspruch 1, wobei der TNF der TNF-α ist.

9. Modifizierter TNF nach Anspruch 1, wobei der TNF ein isolierter humaner TNF ist.

10. Modifizierter TNF nach Anspruch 1, wobei der TNF ein rekombinanter humaner TNF ist.

11. Verfahren zur Steigerung der zirkulierenden Halbwertszeit des TNF, das die Modifizierung des TNF durch kovalente Bindung an zwischen etwa fünf und zwölf PEG Moleküle umfasst, die eine Molekülmasse mit einer ungefähren durchschnittlichen Masse im Bereich von 20.000 und 40.000 aufweisen.

12. Verfahren zur Steigerung der Anti-Tumor Aktivität des TNF, das die Modifizierung des TNF durch kovalente Bindung an zwischen etwa fünf und zwölf PEG Moleküle umfasst, die eine Molekülmasse mit einer ungefähren durchschnittlichen Masse im Bereich von 20.000 und 40.000 aufweisen.

13. Verwendung einer therapeutisch wirksamen Menge des modifizierten TNF nach Anspruch 1 für die Zubereitung einer pharmazeutischen Zusammensetzung zur Behandlung eines an einem Tumor leidenden Patienten.

14. Verwendung nach Anspruch 13, wobei der Tumor Hautkrebs ist.

15. Verwendung nach Anspruch 13, wobei der Tumor Dickdarmkrebs ist.

16. Verwendung nach Anspruch 13, wobei der Tumor Nierenkrebs ist.

17. Verwendung nach Anspruch 13, wobei der Tumor Brustkrebs ist.

## Revendications

1. TNF modifié (facture de nécrose des tumeurs modifié), comprenant le TNF lié de manière covalente à environ entre cinq et douze molécules de PEG ayant un poids moléculaire moyen en poids approximatif compris dans la plage de 20 000 et 40 000.

2. TNF modifié selon la revendication 1, dans lequel ledit PEG a un poids moléculaire moyen en poids approximatif compris dans la plage de 20 000 et 30 000.

3. TNF modifié selon la revendication 1, dans lequel ledit PEG est lié de manière covalente au dit TNF à travers un lieur biocompatible.

4. TNF modifié selon la revendication 1, dans lequel ledit lieur est le succinate de *N*-hydroxysuccinimidyle.

5. TNF modifié selon la revendication 1, dans lequel ledit lieur est le propionate de *N*-succinimidyle.

6. TNF modifié selon la revendication 1, dans lequel ledit TNF est lié de manière covalente à environ cinq à neuf molécules de PEG.

7. TNF modifié selon la revendication 1, dans lequel ledit TNF est lié de manière covalente aux dites molécules de PEG à travers des amines primaires sur le TNF.

8. TNF modifié selon la revendication 1, dans lequel ledit TNF est le TNF-α.

9. TNF modifié selon la revendication 1, dans lequel ledit TNF est le TNF humain isolé.

10. TNF modifié selon la revendication 1, dans lequel ledit TNF est le TNF humain recombinant.

11. Procédé d'amélioration de la demi-vie de circulation du TNF comprenant la modification dudit TNF en lui liant de manière covalente entre environ cinq et douze molécules de PEG ayant un poids moléculaire moyen en poids compris dans la plage de 20 000 à 40 000.

12. Procédé d'amélioration de l'activité tumoricide du TNF comprenant la modification dudit TNF en lui liant de manière covalente entre environ cinq et douze molécules de PEG ayant un poids moléculaire moyen en poids approximatif compris dans la plage de 20 000 à 40 000.

13. Utilisation d'une quantité thérapeutiquement efficace du TNF modifié selon la revendication 1 pour la préparation d'une composition pharmaceutique pour traiter un patient souffrant d'une tumeur.

14. Utilisation selon la revendication 13, dans laquelle ladite tumeur est un mélanome.

15. Utilisation selon la revendication 13, dans laquelle ladite tumeur est un cancer du côlon.

16. Utilisation selon la revendication 13, dans laquelle ladite tumeur est un cancer du rein.

17. Utilisation selon la revendication 13, dans laquelle ladite tumeur est un cancer du sein.
